# EUROPEAN PATENT SPECIFICATION

(11) **EP 3 110 338 B1**
(45) Date of publication and mention of the grant of the patent: **14.08.2019**
(21) Application number: 15708586.1
(22) Date of filing: 22.01.2015
(51) Int. Cl.: A61N 7/00, A61B 17/22, A61B 17/00, A61N 7/02

(54) **SYSTEM FOR PERFORMING INTRALUMINAL HISTOTRIPSY AND METHOD OF OPERATION THEREOF**
SYSTEM ZUR DURCHFÜHRUNG VON INTRALUMINALER HISTOTRIPSIE UND VERFAHREN ZUM BETRIEB DAVON
SYSTÈME POUR LA RÉALISATION D'HISTOTRIPSIE INTRALUMINALE ET PROCÉDÉ DE FONCTIONNEMENT ASSOCIÉ

(30) Priority: 26.02.2014 US 201461944763 P
(43) Date of publication of application: 04.01.2017
(73) Proprietor: Koninklijke Philips N.V., 5656 AE Eindhoven (NL)
(72) Inventor: POPOVIC, Aleksandra, 5656 AE Eindhoven (NL); SEIP, Ralf, 5656 AE Eindhoven (NL); NOONAN, David Paul, 5656 AE Eindhoven (NL)
(74) Representative: Philips Intellectual Property & Standards
(86) International application number: PCT/IB2015/050494
(87) International publication number: WO 2015/128751

(56) References cited:
- WO-A1-2013/150777
- WO-A2-2012/088501
- WO-A2-2012/120495
- US-A1- 2009 230 823

## Description

The present system relates to a system for intraluminally harvesting arteries for surgical procedures and, more particularly, to a system for removing arteries from surrounding connective tissue for harvesting using intraluminal ultrasound techniques, and a method of operation thereof.

Harvesting the left interior mammary artery (LIMA) (also known as the internal thoracic artery, ITA) may be used for coronary-artery bypass-graft surgery, as blood flow from this re-routed vessel is one way to revascularize the myocardium after a myocardial infarction. ITA harvesting is a surgical procedure that requires a highly-skilled surgeon and, can be quite time consuming especially when it is performed using minimally-invasive keyhole surgery methods. For example, ITA harvesting using minimally-invasive keyhole surgery methods can typically require from one to several hours per patient. Recently, this procedure is being performed with the aid of robotic devices (i.e., DaVinci™ device, or other custom vessel harvesting devices, such as harmonic scalpels, electrocautery, or other specialized endoscopic instruments), with some success. These devices typically provide a means of holding the ITA in place during its removal from the chest wall, while further providing means to also cauterize/coagulate ITA side branches to avoid bleeding during the takedown. The skill of the surgeon, however, continues to be a key component of a successful and rapid procedure. WO 2012/120495 A2 discloses a treatment catheter having a flexible body portion having ultrasound transducers situated along a length of the flexible body and being configured to be percutaneously situated in a blood vessel, a controller configured to excite the ultrasound transducers to output ultrasound signals of a first type having a focal zone outside of the vessel walls wherein the ultrasound transducers comprise concave section ultrasound transducers.

The system(s), device(s), method(s), arrangements(s), user interface(s), computer program (s), processes, etc. (hereinafter each of which will be referred to as system, unless the context indicates otherwise), described herein address problems in prior art systems. The invention is defined in claims 1 and 8. Preferred embodiments of the invention are defined in the dependent claims.

In accordance with embodiments of the present system, an apparatus according to the invention enables a method of harvesting a blood vessel, the method performed by an apparatus including a flexible body portion having at least one ultrasound transducer, the apparatus may be controlled by at least one controller, the method may include one or more acts of: percutaneously situating the flexible body into the blood vessel having vessel walls and connective tissue attached to the vessel walls; and exciting the at least one ultrasound transducer to output ultrasound signals of a first type having a focal zone outside of the vessel walls so as to fractionate a region of connective tissue in the focal zone.

It is also envisioned that the method may include an act of exciting the at least one ultrasound transducer to output ultrasound signals of a second type to cauterize side branches of the blood vessel. Further, the method may include an act of forming the ultrasound signals of the first type to include histotripsy pulses and the ultrasound signals of the second type to include high-intensity focused ultrasound (HIFU) pulses that are lower in intensity and longer in duration than the ultrasound signals of the first type. Further, the method may include an act of forming the at least one ultrasound transducer to include one of a spherical-section ultrasound transducer and a truncated-spherical section ultrasound transducer configured to have a focus zone which corresponds with the focal zone. In accordance with yet other embodiments of the apparatus according to the invention, the method may include an act of linearly sweeping the at least one ultrasound transducer so that the fractionated region of connective tissue forms a linear region. It is also envisioned that the method may include an act of rotating the at least one ultrasound transducer so that the fractionated region of connective tissue forms a cylindrical region. The method may further include an act of controlling a positioning mechanism coupled to the flexible body portion to control at least one of position and orientation of at least a portion of the flexible body portion in accordance with position information indicating an in-vivo position of at least a portion of the flexible body portion. The at least one ultrasound transducer may include a plurality of ultrasound transducers arranged across a length of the flexible body portion. Further, it is envisioned that the method may include an act of simultaneously exciting selected ultrasound transducers of the plurality of ultrasound transducers.

In accordance with embodiments of the present system, there is provided an apparatus for harvesting a blood vessel having vessel walls attached to connective tissue. The apparatus may include a flexible body portion having at least one ultrasound transducer situated along a length of the flexible body and being configured to be percutaneously situated in the blood vessel; and a controller configured to excite the at least one ultrasound transducer to output ultrasound signals of a first type having a focal zone outside of the vessel walls so as to fractionate a region of connective tissue in the focal zone. The at least one ultrasound transducer comprises a truncated-spherical section ultrasound transducer configured to have a focus zone which corresponds with the focal zone.

It is further envisioned that the controller may be further configured to excite the at least one ultrasound transducer to further output ultrasound signals of a second type to cauterize side branches of the blood vessel. It is also envisioned that the controller may be further configured to form the ultrasound signals of the first type to include histotripsy pulses and the ultrasound signals of the second type to include high-intensity focused ultrasound (HIFU) pulses that are lower in intensity and longer in duration than the ultrasound signals of the first type. It is further envisioned that the at least one ultrasound transducer may include one of a spherical-section ultrasound transducer and a truncated-spherical section ultrasound transducer configured to have a focus zone which corresponds with the focal zone. Moreover, it is also envisioned that the controller may be further configured to linearly sweep at least a portion of the flexible body portion and the at least one ultrasound transducer attached thereto so that the fractionated region of connective tissue forms a linear region. It is further envisioned that the controller may be further configured to rotate at least a portion of the flexible body portion and the at least one ultrasound transducer attached thereto so that the fractionated region of connective tissue forms a cylindrical region. It is further envisioned that the apparatus may further include a positioning mechanism coupled to the flexible body and which may be configured to control at least one of position and orientation of at least a portion of the flexible body portion and the at least one ultrasound transducer attached thereto. It is also envisioned that the at least one ultrasound transducer may include a plurality of ultrasound transducers arranged across a length of the flexible body portion, and wherein the controller may be further configured to selectively drive individual ones of the ultrasound transducers of the plurality of ultrasound transducers.

In accordance with embodiments of the present system, there is provided a computer program stored on a computer readable non-transitory memory medium, the computer program may be configured to control a flexible apparatus including a flexible body portion having at least one ultrasound transducer, to perform a blood vessel harvesting procedure, the computer program may include a program portion configured to determine at least one of a location and orientation of at least a portion of the flexible body portion when the flexible body portion is situated at least in part within the blood vessel having vessel walls and connective tissue attached to the vessel walls and form corresponding location information; and excite the at least one ultrasound transducer to output ultrasound signals of a first type having a focal zone outside of the vessel walls so as to fractionate a region of connective tissue in the focal zone.

It is also envisioned that the program portion may be further configured to excite the at least one ultrasound transducer to output ultrasound signals of a second type to cauterize side branches of the blood vessel in accordance with the location information obtained in-vivo and relating to at least one of position and orientation of at least a portion of the flexible body portion.

It is further envisioned that the program portion may be further configured to drive the at least one ultrasound transducer to generate the ultrasound signals of the first type to include histotripsy pulses and the ultrasound signals of the second type to include high-intensity focused ultrasound (HIFU) pulses that are lower in intensity and longer in duration than the ultrasound signals of the first type in accordance with the location information. It is also envisioned that the program portion may be further configured to control a positioning mechanism to linearly sweep at least a portion of the flexible body portion and the at least one ultrasound transducer coupled thereto so that the fractionated region of connective tissue forms a linear region. It is further envisioned that the program portion may be further configured to control a positioning mechanism to rotate at least a portion of the flexible body portion and the at least one ultrasound transducer attached thereto so that the fractionated region of connective tissue forms a cylindrical region.

The present invention is explained in further detail in the following exemplary embodiments and with reference to the figures, where identical or similar elements may be partly indicated by the same reference numerals, and the features of various exemplary embodiments being combinable. In the drawings:
FIG. 1 shows a perspective view of portion of flexible tool in accordance with embodiments of the present system;
FIG. 2A shows a detailed cross sectional view of a portion of the ultrasound transducer array of the flexible body situated within an internal thoracic artery (ITA) in accordance with embodiments of the present system;
FIG. 2B shows a detailed cross sectional side view of a portion of the ultrasound transducer array of the flexible body as it is linearly swept to form a linear fractionated tissue volume in accordance with embodiments of the present system;
FIG. 2C shows a cross section of a portion of the ultrasound transducer array of the flexible body taken along lines 2C-2C of FIG. 2B as it is rotationally swept to form a cylindrical fractionated connective tissue volume in accordance with embodiments of the present system;
FIG. 2D shows a cross section of a portion of the ultrasound transducer array of the flexible body with substantially all the connective tissue in a surrounding cylindrical volume fractionated in accordance with embodiments of the present system;
FIG. 3A shows a graph of a spherical-section focused ultrasound transducer in accordance with embodiments of the present system;
FIG. 3B shows a graph of a truncated spherical section focused ultrasound transducer in accordance with embodiments of the present system;
FIG. 4 shows a graph of a simulated pressure distribution map for a single truncated spherical shell ultrasound transducer operating in accordance with embodiments of the present system; and
FIG. 5 shows a portion of a system in accordance with embodiments of the present system.

The following are descriptions of illustrative embodiments that when taken in conjunction with the following drawings will demonstrate the above noted features and advantages, as well as further ones. In the following description, for purposes of explanation rather than limitation, illustrative details are set forth such as architecture, interfaces, techniques, element attributes, etc. However, it will be apparent to those of ordinary skill in the art that other embodiments that depart from these details would still be understood to be within the scope of the appended claims. Moreover, for the purpose of clarity, detailed descriptions of well known devices, circuits, tools, techniques, and methods are omitted so as not to obscure the description of the present system. It should be expressly understood that the drawings are included for illustrative purposes and do not represent the entire scope of the present system. In the accompanying drawings, like reference numbers in different drawings may designate similar elements. Further, in some figures, cross-hatching may be omitted for the sake of clarity. The term and/or and formatives thereof should be understood to mean that only one or more of the recited elements may need to be suitably present (e.g., only one recited element is present, two of the recited elements may be present, etc., up to all of the recited elements may be present) in a system in accordance with the claims recitation and in accordance with one or more embodiments of the present system.

For the sake of clarity, embodiments of the present system will now be discussed with regard to harvesting and/or takedown of a selected portion of the ITA. However, it should be understood that the harvesting and/or takedown methods described may be suitably applied to other vessels (e.g., arteries and/or veins) with similar results.

Embodiments of the present system may provide a system and method to use histotripsy methods (hereinafter histotripsy) to fractionate tissue in accordance with embodiments of the present system. Generally, histotripsy is a tissue fractionation method that relies upon high-pressure and highly-focused ultrasound pulses generated by an ultrasound transducer to emulsify tissue in a non-thermal manner. More particularly, histotripsy relies upon the mechanism of acoustic cavitation to fractionate tissue. As a result of the acoustic cavitation, micro-bubbles generated and maintained by the high-pressure pulses from gases dissolved in the tissue energetically oscillate next to and within tissue, fractionating cells of the tissue in the process. Tissue fractionation occurs only in the focal zone of a corresponding ultrasound transducer, making this process highly localized with sharply demarcated boundaries separating fractionated tissue from tissue that is not fractionated. In bulk tissue, histotripsy produces mechanical fragmentation that results in a liquefied core. Typically, operating parameters to achieve histotripsy in accordance with embodiments of the present system may include one or more of pressures which exceed 10MPa, pulse durations in the µs range, and pulse repetition frequencies in the kHz range.

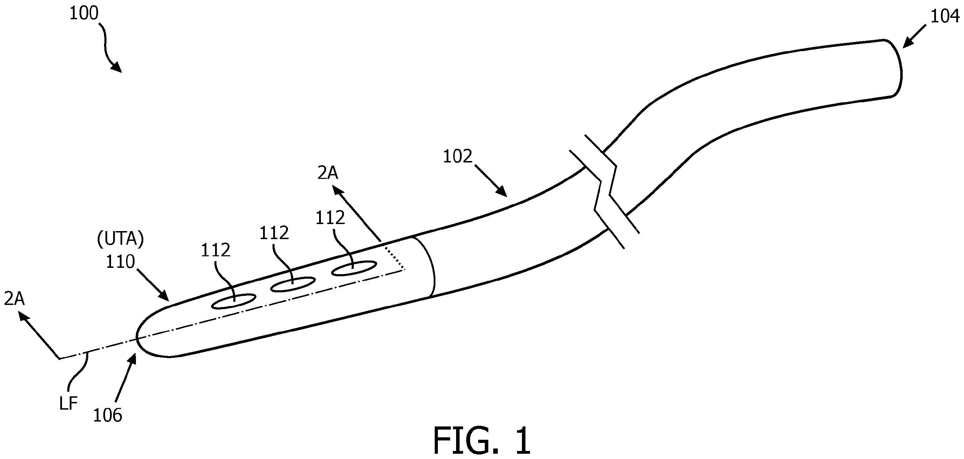

Embodiments of the present system may employ non-thermal tissue fractionation methods such as histotripsy for ITA harvesting and/or takedown as is illustratively described with reference to FIG. 1 which shows a perspective view of a portion of flexible tool 100 in accordance with embodiments of the present system. The flexible tool 100 may include a flexible body 102 having proximal and distal ends 104 and 106, respectively. An ultrasound transducer array (UTA) 110 may be situated at the distal end 106 and may include a plurality of ultrasound transducers 112 (hereinafter transducer 112 for the sake of clarity) which may be driven by a controller (e.g., a processor, logic device, etc.) separately or in unison as may be determined by the controller. The flexible body 102 may be flexible in at least some portions so that it may be percutaneously introduced into a selected vessel (e.g., a blood vessel such as an artery or vein such as the ITA) directly or via a path from an incision/entry point. Accordingly, the flexible body 102 may be configured to resemble a catheter-like instrument. In some embodiments, the flexible tool 100 may include a guide wire which may provide a path for the flexible body 102 to follow. Accordingly, the flexible body 102 may be configured to be coupled to the guide wire, so that it may be guided to a desired location within a desired blood vessel such as the ITA. For example, the guide wire may be positioned leading to a location and the flexible tool 100 may be fed along the guide wire to arrive at the location as is readily appreciated.

Further, in some embodiments, the flexible body 102 may be configured to be inserted from an introduction point which is remote from the ITA such as from an incision/entry point such as in a leg at the femoral artery of the patient. Accordingly, the flexible body 102 may have a suitable length so that it may reach the desired location from the intended insertion point, if desired. In some embodiments, at least a portion of the flexible body 102 may be rigid such as at the UTA 110, if desired and may define a longitudinal axis La for the sake of clarity.

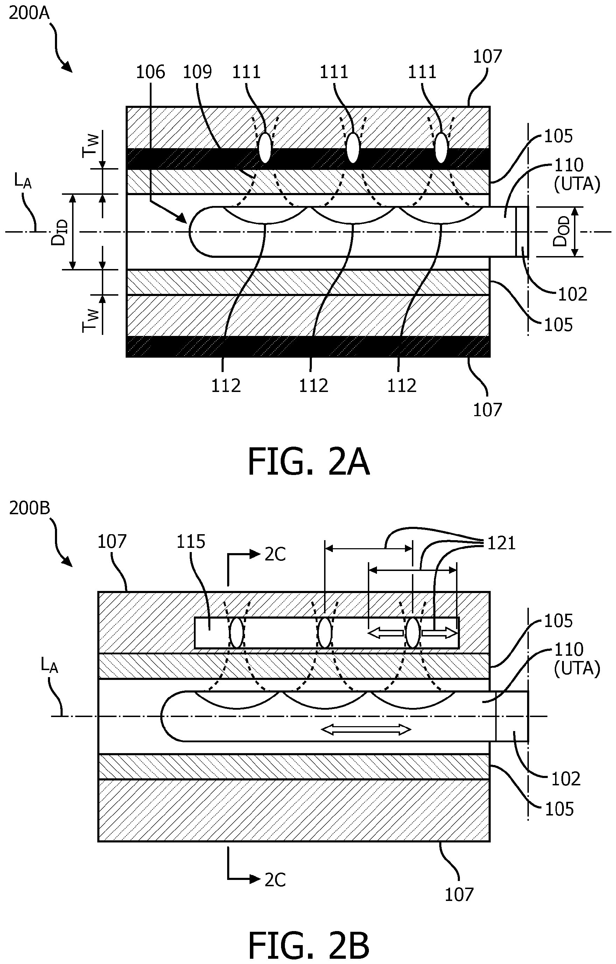

FIG. 2A shows a detailed cross sectional view of a portion of the ultrasound transducer array (UTA) 110 of the flexible body 102 situated within an ITA in accordance with embodiments of the present system. The transducers 112 may include one or more transducers 112 (e.g., a series of transducers 112, such as focused ultrasound transducers) arranged along a longitudinal length of the UTA 110. At least one of the transducers 112 may be electronically coupled to a controller within the flexible body 102 and/or remotely located relative to the flexible body 102. At least one of the transducers 112 may have a spherical and/or truncated spherical section which may focus an ultrasound signal emitted therefrom in a desired focal zone which may have a desired shape, size, and/or location relative to the transducer 112 from which it is emitted. The apparatus of the invention comprises at least one truncated spherical shell ultrasound transducer.

For example, at least one of the transducers 112 may be configured to focus an ultrasound signal 109 at a focal zone 111 that is located beyond an ITA vessel wall 105 so as to fractionate tissue at the focal zone 111. Accordingly, at least one of the transducers 112 may have a shape, size, and/or geometry configured to focus a corresponding ultrasonic signal 109 at the focal zone 111 to fractionate tissue at the focal zone 111. However tissue through which the signal 109 passes and which lies outside of the focal zone 111 (such as the walls of the ITA) are not fractionated (or otherwise damaged) by the ultrasound signal 109. In accordance with embodiments of the present system, the at least one transducers may be configured such that the focal zone 111 is located in an area such that it is within connective tissue 107 which surrounds the ITA. Accordingly, the connective tissue 107 at the focal zone 111 is fractionated and in operation, the transducers may form a corresponding fractionated volume. With regard to the focal zones 111, the shape and size of the ultrasound transducers (e.g., spherical and/or truncated spherical section) may be configured to obtain a desired focal zone shape, size, and/or location of fractionation. Further, the focal zone 111, size and/or shape of one or more of the transducers 111 may be the same as or different from the focal zone 111, size and/or shape of other transducers 112.

In accordance with embodiments of the present system, the controller may drive (e.g., excite) one or more selected transducers 112 of the transducers independently of each other varying one or more of timing of actuation, power, etc. However, in yet other embodiments, the controller may drive all the transducers 112 at the same time, with the same power, etc. In accordance with embodiments of the present system, the controller may drive the transducers to form a continuous, or substantially continuous, fractionated tissue volume, such as a fractionated tissue volume 115 as shown in FIG. 2B, within the connective tissue 107 so that the ITA may be easily separated or removed from at least part of the connective tissue 107 such as a substantial portion of the connective tissue 107. The fractionated tissue volume 115 may be referred to as a weakened area which may separate when, for example, a force is applied to the ITA. For example, in some embodiments, after the connective tissue 107 is fractionated, some of the connective tissue 107 may remain attached to the ITA when the ITA is separated from a substantial portion of the connective tissue 107. However, in yet other embodiments, the connective tissue 105 may be fractionated at or near to where it was originally attached to the ITA. Thus, by changing the geometry, excitation power, etc. of the transducers 112, the focal zone 111 and, thus, at least a portion of the fractionation volume 115 may be tailored as may be desired.

Histotripsy transducers need to be highly focused, in order to achieve the desired histotripsy intensities at their focal zone. In accordance with embodiments of the present system, such devices may have an f/number (i.e. focal length/aperture) of ≤ 1. Coupling of the ultrasound energy from the face of the transducer to its focal zone may be accomplished by the blood and vessel wall. The geometry of this transducer is driven by the inner and outer diameter of the ITA, as its focal zone must always be located outside of the vessel walls. For example, in accordance with embodiments of the present system, a 4x2 mm aperture transducer with a 4 mm radius of curvature will generate a focal zone 4 mm away from its face, placing it outside the ITA and inside the connective tissue. Such a device would fractionate an ellipsoidal volume approximately 1x0.5x0.5 mm in size. Catheter/transducer linear translation and rotation, as described below, yields to larger fractionated volumes for ITA takedown.

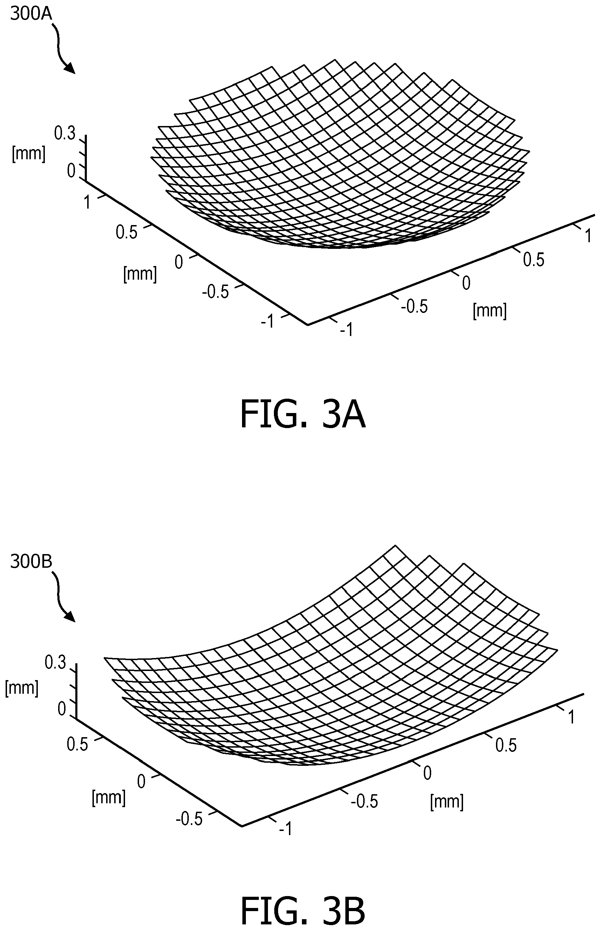

Illustrative geometries of spherical-section focused ultrasound transducers will now be discussed with reference to FIG. 3A which shows a graph 300A of a spherical-section focused ultrasound transducer in accordance with embodiments of the present system; and FIG. 3B which shows a graph 300B of a truncated spherical section focused ultrasound transducer in accordance with embodiments of the present system. The apparatus of the invention comprises at least one truncated spherical shell ultrasound transducer. The truncated spherical-section focused ultrasound transducer may also be referred to as a truncated spherical shell. Thus, in accordance with embodiments of the present system, the transducers 112 may have spherical and/or truncated spherical section ultrasound transducer geometries. One or more of the transducers 112 which include these geometries are capable of generating high acoustic pressures at their focal zones which are suitable for performing histotripsy methods in accordance with embodiments of the present system. As may be readily appreciated, other geometries that are capable of generating high acoustic pressures at their focal zones are suitable for performing histotripsy methods in accordance with embodiments of the present system, however, other geometries (e.g., planar, cylindrical, etc.) that may not have sufficient geometric gain for performing histotripsy methods would not be suitable for application in accordance with embodiments of the present system. Further, as the geometry of the ultrasound transducers may match the geometry of the flexible body 102, they may be easily integrated with the flexible body 102 in accordance with embodiments of the present system.

Thus, by taking into account the blood vessel geometry (e.g., vessel inside diameter, wall thickness (T_{w}), etc.) and the thickness of the connective tissue, the geometries of the flexible body 102 and the transducers 112 may be determined so that one or more of the focal zones 111 of corresponding transducers 112 may be located outside of the vessel wall, but inside the connective tissue 107 that holds the vessel in place. For example, the outside diameter of the flexible body at the UTA should be smaller (e.g., slightly smaller, such as 5% smaller than the inside diameter of the vessel (as defined by the cavity walls of the vessel) so that the transducers are situated proximate to the vessel wall and are still movable within the vessel. Further, a focus zone 111 may be determined in accordance with the thickness of the vessel wall and vessel diameter.

FIG. 2B shows a detailed cross sectional side view of a portion of the ultrasound transducer array (UTA) 110 of the flexible body 102 as it is driven and linearly swept to form a linear fractionated tissue volume 115 in accordance with embodiments of the present system. Once the flexible body 102 is determined to be situated in a desired position within a desired portion of the ITA, the controller may drive selected transducers 112 of the UTA 110 (e.g., individually or simultaneously) to fractionate tissue at focal zones 111 of the corresponding transducers 112 that are driven. Once a portion of the connective tissue 107 which couples the selected portion of the ITA to the chest wall is fractionated (e.g., substantially all of the connective tissue 107), the corresponding portion of the ITA may be removed from the chest wall. During this process, a positioning mechanism under the control of the controller may be configured to slidably move the flexible body 102 (or portions thereof such as the UTA 110) back and forth (e.g., linearly) by a desired distance relative to the ITA so that the focal zones 111 of each of the corresponding transducers 112 may be swept along the ITA so as to form a linear fractionated connective tissue volume 115 along a length of the ITA. For example, in some embodiments, the controller may control the positioning mechanism to slidably move the flexible body 102 (e.g., such as in a back and forth motion) so that the focal zone 111 of each corresponding transducer 112 while being driven (e.g., continuously, periodically, together, separately, etc.) sweeps a distance between its current location and a location of an adjacent focal zone 111 (e.g., of an adjacent transducer 112, which distance may be defined as an intra-transducer distance (ITD) as illustrated by arrows 121). In this way in accordance with embodiments of the present system, the linear region of fractionated connective tissue 115 may be generated. Thus, for example, the back and forth motion of the flexible body 102 may fractionate a linear region of connective tissue 107 so as to form the linear fractionated connective tissue volume 115. In some embodiments, the back and forth motion may be limited to the ITD. However, in yet other embodiments of the present system, the back and for the motion may be smaller or larger than the ITD, as may be desired. In yet other embodiments, a user may manually manipulate the flexible body 102 to move the flexible body 102 back and forth so as to generate the linear region of fractionated connective tissue 115. In yet further embodiments, the controller may receive sensor information indicating linear position and/or orientation of the flexible body 102 and may control the positioning mechanism accordingly so that connective tissue that has not been fractionated may be placed in a focal zone of a transducer 111 for fractionation. The above-mentioned process of moving at least a portion of the flexible body 102 back-and-forth may be referred to as a linear sweeping process. In some embodiments, the UTA may include a plurality of rows and/or columns of ultrasound transducers. Moreover, in some embodiments, the ultrasound transducers in adjacent rows and/or columns may be aligned or slightly offset from each other.

However, in yet other embodiments, sensors (e.g., echo sensors) and/or an external imaging device, (e.g., CT, ultrasound, etc.) may sample or otherwise image connective tissue 107 to detect whether it has been fractionated and provide this information to the controller for further processing. Thus, if it is determined that the sampled connective tissue 107 has not been fractionated, then the controller may control the positioning mechanism to position and/or orientate the flexible body 102 so that the transducers 112 are correctly positioned to fractionate the sampled connective tissue 107. However, if it is determined that the sampled connective tissue 107 has been fractionated, the controller may sample other connective tissue 107 at another location.

In some embodiments the UTA 110 may include a plurality of transducers 112 that may be similar to or different from each other. The UTA 110 may extend any length of the flexible body 102 which may be inserted into the UTA. The location of the flexible body 102 may be determined using any suitable method such as navigation-assisted surgical imaging methods or the like. Then, only those ultrasound transducers which are determined (e.g., by the controller) to be situated within a portion of the ITA that is to be separated from connective tissue attached thereto, are driven by the controller to fractionate the connective tissue. Conversely, those ultrasound transducers which are determined not to be situated within the portion of the ITA that is to be separated are not driven (e.g., remain turned off). Then, once connective tissue in a region that lies around that portion of the ITA selected for harvesting is fractionated, the corresponding portion of the ITA may be separated for harvesting. In accordance with some embodiments, the selected ultrasound transducers may be driven individually (based upon location, time, etc.) or may all be driven at the same time, as desired.

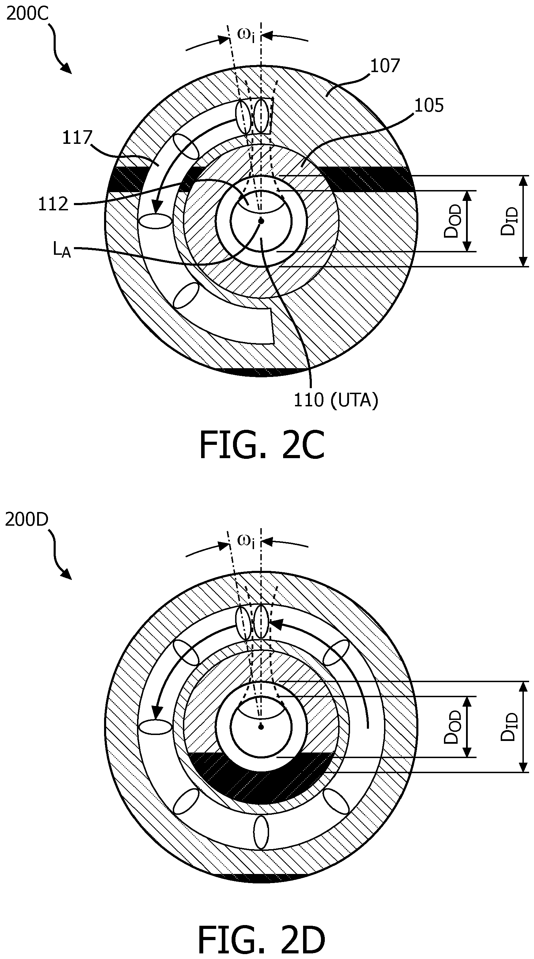

FIG. 2C shows a cross section of a portion of the ultrasound transducer array (UTA) 110 of the flexible body 102 taken along lines 2C-2C of FIG. 2B as it is rotationally swept to form a cylindrical fractionated connective tissue volume 117 in accordance with embodiments of the present system. In accordance with embodiments of the present system, after the linear fractionated connective tissue volume 115 is generated, the flexible body 102 may be rotated (e.g., about its longitudinal axis La) to fractionate a cylindrical region of connective tissue 107 so as to form at least part of a cylindrical fractionated connective tissue volume 117. For example, after a linear fractionated connective tissue volume 115 along a length of the ITA is generated, the flexible body 102 may be rotated by a desired rotational angle (ωᵢ) (about the longitudinal axis (La)) so as to form at least part of a cylindrical fractionated connective tissue volume 117. In accordance with some embodiments, the rotational angle (ωᵢ) may be set such that current and next focal zone locations may overlap. Thus, the controller may control the positioning mechanism to rotate the flexible body 102 by the rotational angle (ωᵢ) so that the focal zones are in a different (adjacent) orientation to a current orientation so that connective tissue that has not exposed to a focal zone 111 (e.g., of histotripsy ultrasound pulses) may be exposed to the focal zone 111 and thus, may be fractionated.

This process of rotating at least a portion of the flexible body 102 back-and-forth may be referred to as a rotational sweeping process. Accordingly, after completing the rotational sweeping process, the process may repeat the linear sweeping process and vice versa. The linear and rotational sweeping processes may be repeated until all (or substantially all) of the connective tissue 107 which couples the selected portion of the ITA to the chest wall is fractionated and the corresponding portion of the ITA may be easily removed from the chest wall. In accordance with some embodiments, the rotational angle (ω) may be set by the system and/or user and may be stored in a memory of the system for later use. Further, in some embodiments, it is envisioned that the rotational angle (ω) may be less than, equal to or greater than 360 degrees depending upon system settings which may be stored in a memory of the system. As may be readily appreciated, depending on the positioning of the connective tissue with regard to the ITA, selected ultrasound transducers may be driven individually (based upon location, time, etc.) or may all be driven at the same time, as desired during all or a portion of the rotation. In accordance with embodiments of the present system, the rotational angle (ω) and the time that the transducers are driven may be selected to ensure that the transducers are only driven when there is connective tissue in the focal zone that it is desired to fractionate. For example, in a case wherein the ITA or other vessel is not attached to the body via connective tissue around its entire periphery, a rotation of 360 degrees or more may not be desired.

In accordance with some embodiments, the flexible body portion may be advanced along a length of the ITA and thereafter the linear and rotational sweeping may be repeated. As may be readily appreciated, rotational sweeping may precede the linear motion (e.g., rotational motion followed by the back and forth motion or simply motion in one direction, such as linear motion in one direction as the flexible body is retracted from an inserted position).

For example, FIG. 2D shows a cross section of a portion of the ultrasound transducer array (UTA) 110 of the flexible body 102 with substantially all the connective tissue 107 in a surrounding cylindrical volume fractionated in accordance with embodiments of the present system. FIG. 2D is similar to FIG. 2C, however, the cylindrical fractionated connective tissue volume 117 is illustratively shown completely surrounding the vessel (e.g., the ITA). Accordingly, the corresponding portion of the ITA is ready for harvesting and may be easily detached from the chest wall. As readily appreciated, to remove the ITA or any other vessel from the chest wall or other related connective tissue, the tissue layers on at least one side of the vessel are fractionated or otherwise detached to enable movement of the vessel to the heart in order to provide the revascularization as described. For example, in a case wherein a cylindrical layer of connective tissue is fractionated, but remains inside a different tissue layer, then the different tissue layer on at least one side of the vessel are fractionated or otherwise detached in accordance with embodiments of the present system.

Further, with regard to the size and shape of the flexible body 102, the flexible body 102 may have a circular cross section with an outside diameter D_{OD} (e.g., see, FIG. 2A) which is slightly smaller than the inside diameter of a flow passage of ITA (D_{ID}) so as to establish a snug fit in which the transducers 112 may be close to an adjacent portion of the ITA wall and the UTA 110 may be self-aligning when situated within the ITA. For example, in some embodiments D_{OD} of the flexible body 102 may correspond with the inner diameter of the ITA (on the order of 3mm) for harvesting the ITA. However, other values, or ranges of values, for the D_{OD} are also envisioned and may be based upon a desired application.

In accordance with embodiments of the present system, after the fractionation process is performed, the fractionation may be tested to see whether the fractionation process was successfully performed. Accordingly, the controller may determine whether the fractionation process has successfully fractionated all the desired connective tissue. For example, the controller may drive the ultrasound transducers and thereafter obtain information from the ultrasound transducers which may be used to determine if the ITA remains attached to the connective tissue. For example, the controller may be operative to drive selected ultrasound transducers as simple pulse-echo devices, to determine the location of the last echo interface (presumably the outer vessel wall in the case of a detached ITA), or the presence of additional scatterers beyond the vessel wall (as would be the case if the ITA is still attached to surrounding connective tissue). Accordingly, if the controller detects the presence of additional scatters beyond the vessel wall (e.g., the ITA wall), the controller may determine to repeat the fractionation process as the fractionation process was not completed successfully. Conversely, if the controller does not detect the presence of additional scatters beyond the vessel wall, the controller may perform the fractionation process on another region of connective tissue as the fractionation process on the current tissue was completed successfully. The controller may repeat this act until all, or a desired portion of, fractionated connective tissue is checked for connective tissue where there should be fractionated tissue.

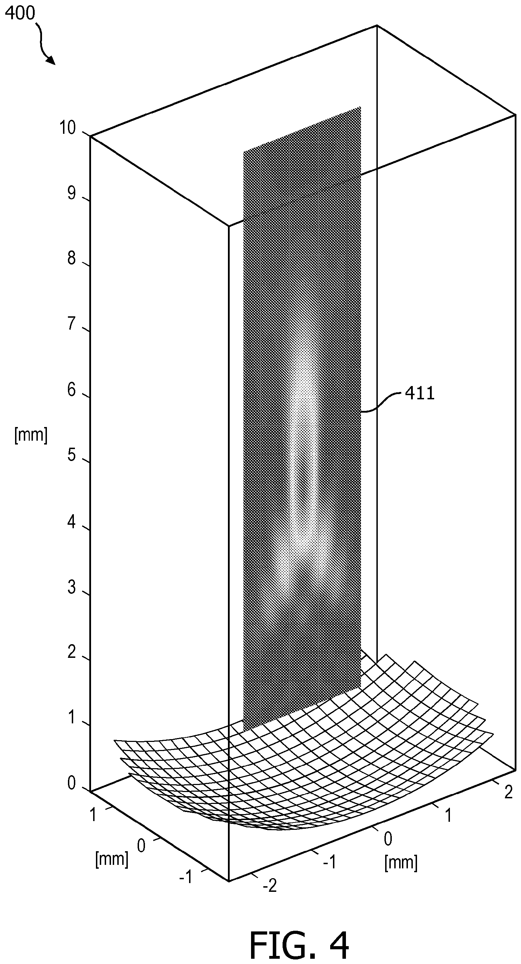

FIG. 4 shows a graph 400 of a simulated pressure distribution map for a single truncated spherical shell ultrasound transducer operating in accordance with embodiments of the present system. A focal zone 411 is shown in the center of the map and corresponds with pressure requirements for performing histotripsy methods in accordance with embodiments of the present system. More particularly, simulations for a 5mm long and 3mm wide truncated spherical shell ultrasound transducer with a focal zone 5mm from its surface (f/number=1) and which operates at 4MHz, indicate that the focal zone size is approximately 2.5mm long and 0.36mm in diameter (e.g., ellipsoidal shaped). Further, it has been found that the above-described ultrasound transducer is capable of generating an intensity of 4.5kW/cm² in its focal zone, which corresponds to a pressure of 11.7MPa (e.g., when being excited by pulses that on a crystal surface of a crystal transducer generate intensities smaller than 25W/cm²). Moreover, these excitation pulses are achievable with current ultrasound transducer crystal technologies. Further, these pressures are capable of delivering high-pressure pulses desired for tissue fractionation using histotripsy methods performed in accordance with embodiments of the present system.

Advantages of ITA harvesting in accordance with embodiments of the present system (where the flexible body may be referred to as a catheter) may include:
(1) Alignment of the catheter and positioning of the individual transducers may be automatically accomplished by the catheter inside the ITA. This is due, at least in part, to the configuration of the ultrasound transducers for example at a distal end of the catheter.
(2) The geometry of the catheter and its ultrasound transducers may be utilized to assure that the focal zone of the transducers is always located outside of the vessel wall, eliminating the possibility of vessel wall (e.g., ITA wall) rupture during the application of the histotripsy pulses.
(3) Histotripsy and tissue fractionation only occur in the focal zone of each corresponding transducer. Everywhere else, the emitted pressures are not sufficient to generate tissue fractionation, thus reducing/eliminating ancillary tissue damage. This "self-limiting" mechanism is produced as a result of the transducer geometry and the level of transducer excitation.
(4) Blood flow around the catheter and transducers may provide for transducer cooling and an automatic coupling mechanism to transfer the ultrasound energy from the transducer to an extraluminal space.
(5) Minimal motion and manipulation of the intraluminal catheter during the procedure simplifies use.
(6) Further, by locating the focal zone a slight distance away from an exterior periphery of the harvested vessel (e.g., ITA), a layer of healthy facia may surround the harvested vessel. This layer of healthy facia may improve the long-term outcome of a coronary bypass procedure.

In accordance with embodiments of the present system, a cauterization process termed a branch vessel management/acoustocautery procedure may be performed as described herein. The cauterization process may acoustically cauterize the ITA side branches (e.g., branch vessels) of the ITA (or other vessel) so as to prevent blood loss from the ITA and may be performed prior to a vessel harvesting procedure/histotripsy procedure described above in which the connective tissue is fractionated and the vessel removed therefrom. After performing the cauterization process, the ITA may be removed from its branch vessels with little or no bleeding. In accordance with embodiments of the present system, rather than delivering the histotripsy pulses, the transducers may deliver lower intensity, but longer duration high-intensity focused ultrasound (HIFU) pulses to cauterize tissue. Accordingly, these HIFU pulses may be used to cauterize blood vessels. This process may be called an acoustocautery process in which blood and tissue, such as the blood contained in ITA side branches and the tissue that forms the ITA side branches, respectively, are thermally coagulated, thus stopping blood flow within the ITA side branches.

In accordance with embodiments of the present system, the ITA side branches may be identified and located using any suitable imaging method or methods such as (extracorporeal) imaging modalities (i.e., X-ray, ultrasound, CT, etc.), navigation-assisted surgical imaging methods or the like. Then using information related to the determined locations of the ITA side branches, the controller may control the positioning mechanism to position and/or orientate the flexible body 102 so that the transducers 112 (or selected ultrasound transducers) are correctly positioned to deliver high-intensity focused ultrasound (HIFU) pulses to cauterize these ITA side branches in accordance with embodiments of the present system. However, in yet other embodiments, it is envisioned that the controller may select transducers 112 that are currently positioned to deliver high-intensity focused ultrasound (HIFU) pulses to cauterize these ITA side branches and then drive these selected ultrasound transducers to deliver the HIFU pulses to cauterize the corresponding ITA side branches in accordance with embodiments of the present system. For example, in some embodiments, once the position of ITA side branches has been identified, the flexible body 102 may be positioned and/or oriented so that at least one of its focused transducers 112 may be oriented towards a corresponding ITA side branch and thereafter activated in accordance with an acoustocautery process of the present system to deliver the HIFU pulses.

With regard to the HIFU pulses, these pulses may generate ultrasound exposures lasting several tens of seconds (e.g., 1-30 seconds) of continuous wave (CW) exposure, at lower intensities (e.g., on the order of 500-2000W/cm²) to coagulate the blood in the corresponding ITA side branch vessel(s). The coagulation may be facilitated even further, as blood flow in the branches (due to the presence of the flexible body 102 in the ITA) is expected to be minimal.

After performing an acoustocautery process to cauterize a side branch to prevent blood flow, the process may check whether there is any blood flow in the cauterized ITA side branches. The process may do this using any suitable method. For example, in accordance with embodiments of the present system, ultrasound Doppler techniques may be employed to detect whether there is any blood flow in an ITA side branch after the acoustocautery process is performed. Accordingly, if it is determined that there is blood flow in the corresponding ITA side branches, the process may repeat (or preform for the first time depending upon embodiments) the acoustocautery process to cauterize the corresponding ITA side branch. However, if it is determined that there is no blood flow in the corresponding ITA side branches (e.g., indicating a successful acoustocautery process), the process may repeat the act of detecting whether there is any blood flow in other ITA side branches and perform the determination act until all the ITA side branches (e.g., along the path of the ITA that is to be removed) have been tested at which point the process may end. In accordance with embodiments of the present system, the ultrasound Doppler techniques may be performed using one or more of the transducers 112.

In accordance with embodiments of the present system, the flexible body may be coupled to an image-guidance system so that the exact position and/or orientation of the flexible body and/or portions thereof such as the ultrasound transducers may be determined in real time in-vivo. In accordance with embodiments of the present system, the flexible body may be coupled to a robotic arm having a plurality of degrees of freedom (e.g., 6 or more degrees of freedom). The robotic arm may be controlled by the controller that controls the transducers and/or may be separately controlled from the transducers. Further, a plurality of sensors may provide information related to position and/or orientation of the flexible body and//or ultrasound transducers in real time and may control the robotic arm to position and/or orientate the flexible body in a desired position and/or orientation. In yet other embodiments, the flexible body may form at least part of a robotic catheter. The robotic catheter may also serve as an actuation mechanism to rotate and/or translate the ultrasound transducers around an arterial wall such as a wall of the ITA.

In accordance with embodiments of the present system, the process may generate a graphical user interface (GUI) which may display a two- or three-dimensional image of the patient including a region of interest obtained using any suitable medical imaging method and may superimpose an image of the flexible device relative to the patient for the convenience of a user (e.g., a surgeon, etc.). The process may then generate and provide a user interface with which the user may interact to change parameters, select settings, and/or at least partially control the flexible device, if desired. Thus, a user may override certain acts and/or processes if desired. Further, the process may generate and/or report (e.g., by rendering, etc.) a current status such as cauterization in process, cauterization successful, histotripsy in process, histotripsy successful, catheter removed, etc. which information may be used by the user and/or other processes to complete other acts. For example, once it is determined that the histotripsy was performed successfully, another process or the user may perform a takedown procedure to remove the ITA from the chest wall for placement in a bypass location.

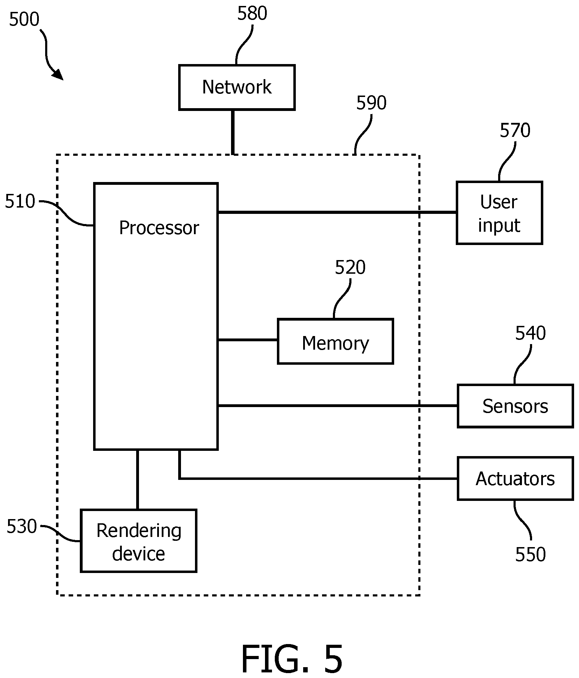

FIG. 5 shows a portion of a system 500 in accordance with embodiments of the present system. For example, a portion of the present system 500 may include a processor 510 (e.g., a controller) operationally coupled to a memory 520, a user interface 530 (e.g., a display), sensors 540, actuators 550, and a user input device 570. The memory 520 may be any type of device for storing application data as well as other data related to the described operation. The application data and other data are received by the processor 510 for configuring (e.g., programming) the processor 510 to perform operation acts in accordance with the present system. The processor 510 so configured becomes a special purpose machine particularly suited for performing in accordance with embodiments of the present system. The sensors may include sensors of a catheter in accordance with embodiments of the present system. For example, the sensors may include imaging sensors, position sensors (e.g., linear, rotational, deflection, etc.), temperature sensors, pressure sensors, echo sensors, flow sensors, status sensors, etc., each of which may provide corresponding information to the controller 510 for further processing.

The operation acts may include configuring the system 500 by, for example, configuring the processor 510 to obtain information from user inputs, the sensors 540, and/or the memory 520 and processing this information in accordance with embodiments of the present system to obtain information related to use of the catheter in accordance with embodiments of the present system. The user input portion 570 may include a keyboard, a mouse, a trackball, rotational wheels, a joystick, and/or other device, including touch-sensitive displays, which may be stand alone or be a part of a system, such as part of a personal computer, a notebook computer, a netbook, a tablet, a smart phone, a personal digital assistant (PDA), a mobile phone, and/or other device for communicating with the processor 510 via any operable link. The user input portion 570 may be operable for interacting with the processor 510 including enabling interaction within a UI as described herein. Clearly the processor 510, the memory 520, the UI 530, the actuators 550, and/or user input device 570 may all or partly be a portion of a computer system or other device as described herein.

Operation acts may include requesting, providing, and/or rendering of information such as, for example, information related to navigation-assisted surgical imaging methods to determine a location of one or more portions of the catheter within a patient during surgery. The navigation-assisted surgical imaging methods may further provide a two- or three-dimensional image of the user using any suitable imaging method or methods such as computer tomography (CT) scanning, X-ray imaging, magnetic resonance imaging (MRI), ultrasound imaging, and the like. The processor 510 may render the information on the UI 530 such as on a display of the system such as status information, image information (e.g., in real-time) which may include images of a region of interest. The sensors may include suitable sensors to provide desired sensor information to the processor 510 for further processing in accordance with embodiments of the present system.

The methods of the present system are particularly suited to be carried out by processor programmed by a computer software program, such program containing modules corresponding to one or more of the individual steps or acts described and/or envisioned by the present system.

The processor 510 is operable for providing control signals and/or performing operations in response to input signals from the user input device 570 as well as in response to other devices of a network and executing instructions stored in the memory 520. For example, the processors 510 may obtain feedback information from the sensors 540 and may process this information to position, orientation, and/or otherwise adjust and/or report a status of portions of the catheter. The processor 510 may determine actions to perform in accordance with embodiments of the present system. The processor 510 may control the actuators to perform corresponding actions. The actuators may include motors (e.g., linear, rotational, etc.), which may control a position and/or orientation of the flexible body under the control of the processor 510. The processor 510 may include one or more of a microprocessor, an application-specific or general-use integrated circuit(s), and/or a logic device, etc. Further, the processor 510 may be a dedicated processor for performing in accordance with the present system or may be a general-purpose processor wherein only one of many functions operates for performing in accordance with the present system. The processor 510 may operate utilizing a program portion, multiple program segments, and/or may be a hardware device utilizing a dedicated or multi-purpose integrated circuit.

While the present invention has been shown and described with reference to particular exemplary embodiments, it will be understood by those skilled in the art that present invention is not limited thereto, but that various changes in form and details, including the combination of various features and embodiments, may be made therein without departing from the scope of the invention as defined by the claims. For example, while the present system is described with regard to the ITA, it may also be suitably applied to other vessels in accordance with embodiments of the present system.

Thus, embodiments of the present system may simplify vessel harvesting (e.g., ITA harvesting) by providing a system and method to remove the selected vessel such as an artery from surrounding connective tissue; and manage vessel side branches (e.g., smaller vessels) to prepare the vessel for bypass graft surgery. Further, embodiments of the present system may perform the vessel harvesting and/or takedown under the control of a controller with little or no user intervention required. In accordance with embodiments of the present system, this automation may reduce the load on users such as surgeons during surgery and may reduce the time required for performing coronary bypass procedures and thereby, may improve outcomes in such procedures. Moreover, embodiments of the present system may provide for coronary artery bypass graft surgery using minimally-invasive methods which may decrease both patient recovery times and patient morbidity rates.

Although embodiments of the present system are shown and described with reference to LIMA/ITA harvesting/takedown for coronary artery bypass graft surgery, it should be appreciated that embodiments of the present system may be used to perform other minimally-invasive vessel-harvesting procedures such as saphenous vein harvesting, etc. In accordance with embodiments of the present system, the diameter of the flexible tool may be selected/designed/constructed to correspond with the diameter of the artery or vein selected for harvesting/takedown. For example, to perform saphenous vein harvesting, the diameter and/or size of the catheter including the ultrasonic transducer array may be changed accordingly so that it may be suitably applied considering the diameter of the saphenous vein.

In accordance with embodiments of the present system, the flexible body may be configured as a single use/disposable type catheter, and may be removed from the patient once the vessel harvesting procedure has been completed, for example to allow for placement and manipulation of other intra-luminal devices to complete a coronary artery bypass graft surgery which may use a vessel harvested in accordance with embodiments of the present system as a graft vessel. However, in yet other embodiments, the flexible body may incorporate other functionalities such as a laser arteriectomy device, etc., as may be desired to complete or otherwise perform other portions of a procedure.

Further variations of the present system would readily occur to a person of ordinary skill in the art and are encompassed by the following claims.

Finally, the above-discussion is intended to be merely illustrative of the present system and should not be construed as limiting the appended claims to any particular embodiment or group of embodiments. Thus, while the present system has been described with reference to exemplary embodiments, it should also be appreciated that numerous modifications and alternative embodiments may be devised by those having ordinary skill in the art without departing from the broader and intended scope of the present system as set forth in the claims that follow. Accordingly, the specification and drawings are to be regarded in an illustrative manner and are not intended to limit the scope of the appended claims.

In interpreting the appended claims, it should be understood that:
a) the word "comprising" does not exclude the presence of other elements or acts than those listed in a given claim;
b) the word "a" or "an" preceding an element does not exclude the presence of a plurality of such elements;
c) any reference signs in the claims do not limit their scope;
d) several "means" may be represented by the same item or hardware or software implemented structure or function;
e) any of the disclosed elements may be comprised of hardware portions (e.g., including discrete and integrated electronic circuitry), software portions (e.g., computer programming), and any combination thereof;
f) hardware portions may be comprised of one or both of analog and digital portions;
g) any of the disclosed devices or portions thereof may be combined together or separated into further portions unless specifically stated otherwise;
h) no specific sequence of acts or steps is intended to be required unless specifically indicated; and
i) the term "plurality of" an element includes two or more of the claimed element, and does not imply any particular range of number of elements; that is, a plurality of elements may be as few as two elements, even when more than two elements make up the plurality and may include an immeasurable number of elements.

## Claims

1. An apparatus (100, 500) for harvesting a blood vessel having vessel walls (105) attached to connective tissue (107), the apparatus comprising:
a flexible body portion (102) having at least one ultrasound transducer (112) situated along a length of the flexible body and being configured to be percutaneously situated in the blood vessel; and
a controller (510) configured to excite the at least one ultrasound transducer to output ultrasound signals of a first type having a focal zone (111) outside of the vessel walls so as to fractionate a region of connective tissue in the focal zone;
wherein the at least one ultrasound transducer (112) comprises a truncated spherical shell ultrasound transducer configured to have a focus zone which corresponds with the focal zone, wherein the truncated spherical shell comprises a portion of the minor spherical surface defined by an intersection between a plane and a sphere, the spherical surface having orthogonal length and width dimensions on the plane (300A, 300B), the width dimension being truncated such that the length dimension exceeds the width dimension (300B) in order to provide the portion, the truncated spherical shell ultrasound transducer being arranged on the flexible body portion (102) such that the length dimension is parallel to the length of the flexible body.

2. The apparatus of claim 1, wherein the controller is further configured to excite the at least one ultrasound transducer to further output ultrasound signals of a second type to cauterize side branches of the blood vessel.

3. The apparatus of claim 2, wherein the controller is further configured to form the ultrasound signals of the first type to comprise histotripsy pulses and the ultrasound signals of the second type to comprise high-intensity focused ultrasound (HIFU) pulses that are lower in intensity and longer in duration than the ultrasound signals of the first type.

4. The apparatus of claim 1, wherein the controller is further configured to linearly sweep at least a portion of the flexible body portion and the at least one ultrasound transducer attached thereto so that the fractionated region of connective tissue forms a linear region (115).

5. The apparatus of claim 1, wherein the controller is further configured to rotate at least a portion of the flexible body portion and the at least one ultrasound transducer attached thereto so that the fractionated region of connective tissue forms a cylindrical region (117).

6. The apparatus of claim 1, further comprising a positioning mechanism coupled to the flexible body and configured to control at least one of position and orientation of at least a portion of the flexible body portion and the at least one ultrasound transducer attached thereto.

7. The apparatus of claim 1, wherein the at least one ultrasound transducer comprises a plurality of ultrasound transducers arranged across a length of the flexible body portion, and wherein the controller is further configured to selectively drive individual ones of the ultrasound transducers of the plurality of ultrasound transducers.

8. A computer program stored on a computer readable non-transitory memory medium (520), the computer program configured to control a flexible apparatus (100) comprising a flexible body portion (102) having at least one ultrasound transducer (112) situated along a length of the flexible body and being configured to be percutaneously situated in the blood vessel, to perform a blood vessel harvesting procedure, the computer program comprising:
a program portion configured to:
determine at least one of a location and orientation of at least a portion of the flexible body portion when the flexible body portion is situated at least in part within the blood vessel having vessel walls (105) and connective tissue (107) attached to the vessel walls and form corresponding location information; and
excite the at least one ultrasound transducer to output ultrasound signals of a first type having a focal zone (111) outside of the vessel walls so as to fractionate a region of connective tissue in the focal zone;
wherein the at least one ultrasound transducer (112) comprises a truncated spherical shell ultrasound transducer configured to have a focus zone which corresponds with the focal zone, wherein the truncated spherical shell comprises a portion of the minor spherical surface defined by an intersection between a plane and a sphere, the spherical surface having orthogonal length and width dimensions on the plane (300A), the width dimension being truncated such that the length dimension exceeds the width dimension in order to provide the portion, the truncated spherical shell ultrasound transducer being arranged on the flexible body portion (102) such that the length dimension is parallel to the length of the flexible body

9. The computer program of claim 8, wherein the program portion is further configured to excite the at least one ultrasound transducer to output ultrasound signals of a second type to cauterize side branches of the blood vessel in accordance with the location information.

10. The computer program of claim 9, wherein the program portion is further configured to drive the at least one ultrasound transducer to generate the ultrasound signals of the first type to comprise histotripsy pulses and the ultrasound signals of the second type to comprise high-intensity focused ultrasound (HIFU) pulses that are lower in intensity and longer in duration than the ultrasound signals of the first type in accordance with the location information.

11. The computer program of claim 8, wherein the program portion is further configured to control a positioning mechanism to move at least a portion of the flexible body portion and the at least one ultrasound transducer coupled thereto so that the fractionated region of connective tissue forms at least one of a linear region (115) and a cylindrical region (117).

## Patentansprüche

1. Vorrichtung (100, 500) zur Gewinnung eines Blutgefäßes mit Gefäßwänden (105), die an Bindegewebe (107) befestigt sind, wobei die Vorrichtung umfasst:
einen flexiblen Körperabschnitt (102) mit mindestens einem Ultraschallwandler (112), der entlang einer Länge des flexiblen Körpers angeordnet ist und konfiguriert ist, um perkutan in dem Blutgefäß angeordnet zu sein; und
eine Steuerung (510), die konfiguriert ist, um den mindestens einen Ultraschallwandler anzuregen, um Ultraschallsignale eines ersten Typs mit einer Fokuszone (111) außerhalb der Gefäßwände auszugeben, um so einen Bereich von Bindegewebe in der Fokuszone zu fraktionieren;
wobei der mindestens eine Ultraschallwandler (112) einen Ultraschallwandler mit verkürzter Kugelschale umfasst, der konfiguriert ist, um einen Fokusbereich aufzuweisen, der der Fokuszone entspricht, wobei die verkürzte Kugelschale einen Abschnitt der kleinen kugelförmigen Oberfläche umfasst, der durch einen Schnitt zwischen einer Ebene und einer Kugel definiert ist, wobei die kugelförmige Oberfläche orthogonale Längen- und Breitenmaße in der Ebene (300A, 300B) aufweist, wobei das Breitenmaß so verkürzt ist, dass das Längenmaß das Breitenmaß (300B) überschreitet, um den Abschnitt bereitzustellen, wobei der Ultraschallwandler mit verkürzter Kugelschale so auf dem flexiblen Körperabschnitt (102) angeordnet ist, dass das Längenmaß parallel zur Länge des flexiblen Körpers ist.

2. Vorrichtung nach Anspruch 1, wobei die Steuerung weiter konfiguriert ist, um den mindestens einen Ultraschallwandler anzuregen, um weiter Ultraschallsignale einer zweiten Art auszugeben, um Seitenäste des Blutgefäßes zu kauterisieren.

3. Vorrichtung nach Anspruch 2, wobei die Steuerung weiter konfiguriert ist, um die Ultraschallsignale des ersten Typs zu bilden, um Histotripsie-Impulse und die Ultraschallsignale des zweiten Typs zu umfassen, um hochintensive fokussierte Ultraschallimpulse (HIFU) zu umfassen, die niedriger in der Intensität und länger in der Dauer sind als die Ultraschallsignale des ersten Typs.

4. Vorrichtung nach Anspruch 1, wobei die Steuerung weiter konfiguriert ist, um mindestens einen Teil des flexiblen Körperabschnitts und den mindestens einen daran befestigten Ultraschallwandler linear zu durchsuchen, so dass der fraktionierte Bereich des Bindegewebes einen linearen Bereich (115) bildet.

5. Vorrichtung nach Anspruch 1, wobei die Steuerung weiter konfiguriert ist, um mindestens einen Abschnitt des flexiblen Körperabschnitts und den mindestens einen daran befestigten Ultraschallwandler zu drehen, so dass der fraktionierte Bereich des Bindegewebes einen zylindrischen Bereich (117) bildet.

6. Vorrichtung nach Anspruch 1, weiter umfassend einen Positionierungsmechanismus, der mit dem flexiblen Körper gekoppelt und konfiguriert ist, um mindestens eine von Position und Ausrichtung mindestens eines Teils des flexiblen Körperabschnitts und des mindestens einen daran befestigten Ultraschallwandlers zu steuern.

7. Vorrichtung nach Anspruch 1, wobei der mindestens eine Ultraschallwandler eine Vielzahl von Ultraschallwandlern umfasst, die über eine Länge des flexiblen Körperabschnitts angeordnet sind, und wobei die Steuerung weiter konfiguriert ist, um einzelne der Ultraschallwandler der Vielzahl von Ultraschallwandlern selektiv anzusteuern.

8. Computerprogramm, das auf einem computerlesbaren, nichtflüchtigen Speichermedium (520) gespeichert ist, wobei das Computerprogramm konfiguriert ist, um eine flexible Vorrichtung (100) zu steuern, die einen flexiblen Körperabschnitt (102) mit mindestens einem Ultraschallwandler (112) umfasst, der sich entlang einer Länge des flexiblen Körpers befindet und konfiguriert ist, um perkutan im Blutgefäß angeordnet zu sein, um ein Verfahren zur Gewinnung von Blutgefäßen durchzuführen, wobei das Computerprogramm umfasst:
einen Programmabschnitt, der konfiguriert ist, um:
mindestens einen von einem Standort und einer Ausrichtung mindestens eines Abschnitts des flexiblen Körperabschnitts zu bestimmen, wenn sich der flexible Körperabschnitt mindestens teilweise innerhalb des Blutgefäßes befindet, das Gefäßwände (105) und Bindegewebe (107) aufweist, die an den Gefäßwänden befestigt sind und entsprechende Standortinformationen bilden; und
den mindestens einen Ultraschallwandler zum Ausgeben von Ultraschallsignalen eines ersten Typs mit einer Fokuszone (111) außerhalb der Gefäßwände anzuregen, um so einen Bereich des Bindegewebes in der Fokuszone zu fraktionieren;
wobei der mindestens eine Ultraschallwandler (112) einen Ultraschallwandler mit verkürzter Kugelschale umfasst, der konfiguriert ist, um einen Fokusbereich aufzuweisen, der der Fokuszone entspricht, wobei die verkürzte Kugelschale einen Abschnitt der kleinen kugelförmigen Oberfläche umfasst, der durch einen Schnitt zwischen einer Ebene und einer Kugel definiert ist, wobei die kugelförmige Oberfläche orthogonale Längen- und Breitenmaße in der Ebene (300A) aufweist, wobei das Breitenmaß so verkürzt ist, dass das Längenmaß das Breitenmaß überschreitet, um den Abschnitt bereitzustellen, wobei der Ultraschallwandler mit verkürzter Kugelschale so auf dem flexiblen Körperabschnitt (102) angeordnet ist, dass das Längenmaß parallel zur Länge des flexiblen Körpers ist.

9. Computerprogramm nach Anspruch 8, wobei der Programmabschnitt weiter konfiguriert ist, um den mindestens einen Ultraschallwandler anzuregen, um Ultraschallsignale einer zweiten Art auszugeben, um Seitenäste des Blutgefäßes gemäß den Standortinformationen zu kauterisieren.

10. Computerprogramm nach Anspruch 9, wobei der Programmabschnitt weiter konfiguriert ist, um den mindestens einen Ultraschallwandler anzusteuern, um die Ultraschallsignale des ersten Typs zu erzeugen, um Histotripsie-Impulse zu umfassen, und die Ultraschallsignale des zweiten Typs, um hochintensive fokussierte Ultraschallimpulse (HIFU-Impulse) zu umfassen, die niedriger in der Intensität und länger in der Dauer sind als die Ultraschallsignale des ersten Typs gemäß den Standortinformationen.

11. Computerprogramm nach Anspruch 8, wobei der Programmabschnitt weiter konfiguriert ist, um einen Positionierungsmechanismus zu steuern, um mindestens einen Abschnitt des flexiblen Körperabschnitts und den mindestens einen damit gekoppelten Ultraschallwandler zu bewegen, so dass der fraktionierte Bereich des Bindegewebes mindestens einen von einem linearen Bereich (115) und einem zylindrischen Bereich (117) bildet.

## Revendications

1. Appareil (100, 500) de récolte d'un vaisseau sanguin présentant des parois de vaisseau (105) attachées à un tissu conjonctif (107), l'appareil comprenant :
une portion de corps souple (102) présentant au moins un transducteur à ultrasons (112) situé le long d'une longueur du corps souple et étant configuré pour être situé de manière percutanée dans le vaisseau sanguin ; et
un élément de commande (510) configuré pour exciter l'au moins un transducteur à ultrasons afin de sortir des signaux à ultrasons d'un premier type présentant une zone focale (111) en dehors des parois de vaisseau de sorte à fractionner une région de tissu conjonctif dans la zone focale ;
dans lequel l'au moins un transducteur à ultrasons (112) comprend un transducteur à ultrasons à coque sphérique tronquée configuré pour présenter une zone focale qui correspond à la zone focale, dans lequel la coque sphérique tronquée comprend une portion de la surface sphérique mineure définie par une intersection entre un plan et une sphère, la surface sphérique présentant des dimensions de longueur et de largeur orthogonales sur le plan (300A, 300B), la dimension de largeur étant tronquée de sorte que la dimension de longueur excède la dimension de largeur (300B) afin de fournir la portion, le transducteur à ultrasons à coque sphérique tronquée étant agencé sur la portion de corps souple (102) de sorte que la dimension de longueur soit parallèle à la longueur du corps souple.

2. Appareil selon la revendication 1, dans lequel l'élément de commande est en outre configuré pour exciter l'au moins un transducteur à ultrasons afin de sortir en outre des signaux à ultrasons d'un second type pour cautériser des branches latérales du vaisseau sanguin.

3. Appareil selon la revendication 2, dans lequel l'élément de commande est en outre configuré pour former les signaux à ultrasons du premier type afin de comprendre des pulsations d'histotripsie et les signaux à ultrasons du second type afin de comprendre des pulsations à ultrasons focalisés à haute intensité (HIFU) qui sont inférieures en intensité et plus longues en durée que les signaux à ultrasons du premier type.

4. Appareil selon la revendication 1, dans lequel l'élément de commande est en outre configuré pour balayer linéairement au moins une portion de la portion de corps souple et l'au moins un transducteur à ultrasons attaché à celle-ci de sorte que la région fractionnée de tissu conjonctif forme une région linéaire (115).

5. Appareil selon la revendication 1, dans lequel l'élément de commande est en outre configuré pour faire tourner au moins une portion de la portion de corps souple et l'au moins un transducteur à ultrasons attaché à celle-ci de sorte que la région fractionnée de tissu conjonctif forme une région cylindrique (117).

6. Appareil selon la revendication 1, comprenant en outre un mécanisme de positionnement couplé au corps souple et configuré pour commander au moins une parmi la position et l'orientation d'au moins une portion de la portion de corps souple et l'au moins un transducteur à ultrasons attaché à celle-ci.

7. Appareil selon la revendication 1, dans lequel l'au moins un transducteur à ultrasons comprend une pluralité de transducteurs à ultrasons agencés sur une longueur de la portion de corps souple, et dans lequel l'élément de commande est en outre configuré pour entraîner sélectivement des individuels des transducteurs à ultrasons de la pluralité de transducteurs à ultrasons.

8. Programme informatique stocké sur un support de mémoire non transitoire lisible sur ordinateur (520), le programme informatique étant configuré pour commander un appareil souple (100) comprenant une portion de corps souple (102) présentant au moins un transducteur à ultrasons (112) situé le long d'une longueur du corps souple et étant configuré pour être situé par voie percutanée dans le vaisseau sanguin, pour réaliser une procédure de récolte de vaisseau sanguin, le programme informatique comprenant :
une portion de programme configurée pour :
déterminer au moins un d'un emplacement et d'une orientation d'au moins une portion de la portion de corps souple lorsque la portion de corps souple est située au moins en partie dans le vaisseau sanguin présentant des parois de vaisseau (105) et du tissu conjonctif (107) attaché aux parois de vaisseau et former des informations d'emplacement correspondantes ; et
exciter l'au moins un transducteur à ultrasons pour sortir des signaux à ultrasons d'un premier type présentant une zone focale (111) en dehors des parois de vaisseau de sorte à fractionner une région de tissu conjonctif dans la zone focale ;
dans lequel l'au moins un transducteur à ultrasons (112) comprend un transducteur à ultrasons à coque sphérique tronquée configuré pour présenter une zone focale qui correspond à la zone focale, dans lequel la coque sphérique tronquée comprend une portion de la surface sphérique mineure définie par une intersection entre un plan et une sphère, la surface sphérique présentant des dimensions de longueur et de largeur orthogonales sur le plan (300A), la dimension de largeur étant tronquée de sorte que la dimension de longueur excède la dimension de largeur afin de fournir la portion, le transducteur à ultrasons à coque sphérique tronquée étant agencé sur la portion de corps souple (102) de sorte que la dimension de longueur soit parallèle à la longueur du corps souple.

9. Programme informatique selon la revendication 8, dans lequel la portion de programme est en outre configurée pour exciter l'au moins un transducteur à ultrasons afin de sortir des signaux à ultrasons d'un second type pour cautériser des branches latérales du vaisseau sanguin selon les informations d'emplacement.

10. Programme informatique selon la revendication 9, dans lequel la portion de programme est en outre configurée pour entraîner l'au moins un transducteur à ultrasons afin de générer les signaux à ultrasons du premier type pour comprendre des pulsations d'histotripsie et les signaux à ultrasons du second type pour comprendre des pulsations à ultrasons focalisés à haute intensité (HIFU) qui sont inférieures en intensité et plus longues en durée que les signaux à ultrasons du premier type selon les informations d'emplacement.

11. Programme informatique selon la revendication 8, dans lequel la portion de programme est en outre configurée pour commander un mécanisme de positionnement afin de déplacer au moins une portion de la portion de corps souple et l'au moins un transducteur à ultrasons couplé à celle-ci de sorte que la région fractionnée de tissu conjonctif forme au moins une d'une région linéaire (115) et d'une région cylindrique (117).
